(19) Europäisches Patentamt
European Patent Office
Office européen des brevets

(11) Numéro de publication: **0 045 709**
**B1**

(12) # FASCICULE DE BREVET EUROPEEN

(45) Date de publication du fascicule du brevet: **03.10.84**

(51) Int. Cl.³: **C 07 C 85/11**, C 07 C 85/24, C 07 C 87/60

(21) Numéro de dépôt: **81420115.8**

(22) Date de dépôt: **30.07.81**

(54) **Procédé de préparation sélective d'anilines métachlorées.**

(30) Priorité: **01.08.80 FR 8017324**
**26.09.80 FR 8020979**

(43) Date de publication de la demande:
**10.02.82 Bulletin 82/6**

(45) Mention de la délivrance du brevet:
**03.10.84 Bulletin 84/40**

(84) Etats contractants désignés:
**AT BE CH DE FR GB IT LI LU NL SE**

(56) Documents cités:
**EP - A - 0 015 219**
**EP - A - 0 015 220**
**FR - A - 2 298 531**

**CHEMICAL ABSTRACTS, vol. 85, no. 19, 8 novembre 1976, réf. no. 142781j, page 496 Columbus, Ohio, US**

(73) Titulaire: **RHONE-POULENC AGROCHIMIE, 14-20, rue Pierre Baizet, F-69009 Lyon (FR)**

(72) Inventeur: **Cordier, Georges, 50 Chemin des Hermières, F-69340 Francheville (FR)**
Inventeur: **Fouilloux, Pierre, 22 bis, avenue Général Leclerc, F-69300 Caluire (FR)**

(74) Mandataire: **Chrétien, François et al, RHONE-POULENC AGROCHIMIE BP 9163, F-69263 Lyon Cedex 1 (FR)**

**EP 0 045 709 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention se rapporte à un procédé de préparation d'anilines substituées par le chlore en position méta par action de l'hydrogène sur des composés aromatiques azotés plus fortement halogénés. Ces anilines métachlorées sont des intermédiaires notamment pour la fabrication de produits phytosanitaires.

La préparation de chloranilines substituées en position méta par réaction de polychloranilines en milieu acide notamment aqueux avec de l'hydrogène sous pression, en présence d'un catalyseur à base de métal noble, a été décrite dans le brevet français 2 298 531. Le procédé décrit nécessite toutefois l'utilisation de pressions élevées et de quantités très importantes d'acide chlorhydrique, ce qui pose de graves problèmes de corrosion. Les demandes de brevet européen 0 015 219 et 0 015 220 décrivent des procédés de préparation d'anilines chlorées en méta par hydrodéchloration catalytique en milieu aqueux acide, en présence de métal noble, de polychloranilines ou polychlorobenzènes plus fortement chlorés, à des pressions toujours supérieures à 3 atmosphères et de préférence à 5 atmosphères.

La présente invention a précisément pour but de préparer des chloranilines substituées en méta par hydrodéchloration sélective de polychloranilines ou polychlorobenzènes, sans problème de corrosion.

L'invention concerne plus particulièrement un procédé de préparation d'anilines substituées en position méta par du chlore, par hydrogénation catalytique en phase liquide, à chaud à la pression atmosphérique ou sous pression, en présence de métaux nobles du groupe VIII de la classification périodique, de dérivés benzéniques azotés et chlorés, de formule:

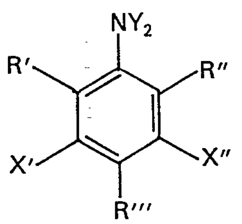

(I)

dans laquelle:

- Y représente l'atome d'hydrogène ou l'atome d'oxygène,
- X′ et X″, identiques ou différents entre eux, représentent chacun un atome de chlore ou un radical alkyle ou aryle ou aralkyle ou alkoxyle ou aralkoxyle éventuellement substitué, au moins l'un des X′ et X″ étant obligatoirement un atome de chlore, l'un des X′ et X″ pouvant, de plus, être l'hydrogène,
- F′, R″ et R‴, identiques ou différents entre eux, représentent chacun un atome de chlore ou un radical alkyle ou aryle ou aralkyle ou alkoxyle ou aryloxyle éventuellement substitué, l'un au moins de ces trois symboles représentant l'atome de chlore, au plus deux des R′, R″ ou R‴ pouvant, de plus, être l'hydrogène,

caractérisé en ce que la réaction est effectuée en milieu essentiellement anhydre, en présence d'une quantité catalytique d'au moins un acide de Lewis comme catalyseur.

Par «acide de Lewis», on entend des composés ayant un atome central déficitaire en électrons captant les électrons d'atomes tels que les halogènes. Les composés préférés sont des chlorures, bromures, iodures ou même fluorures d'éléments tels que le bore, l'aluminium, le gallium, l'étain, le phosphore, l'arsenic, l'antimoine, le bismuth, le titane, le zirconium, le molybdène, le manganèse, le fer, le cobalt, le nickel, le cuivre, le zinc et le cadmium.

La demanderesse a notamment observé qu'on obtient des résultats intéressants avec, comme catalyseur, les chlorure et bromure d'aluminium, les chlorures stanneux et stannique, le chlorure ferrique, les chlorure, bromure et iodure de zinc, le chlorure cuivreux et le chlorure de nickel, des résultats particulièrement avantageux étant obtenus en utilisant le chlorure ou le bromure d'aluminium.

Par quantité catalytique, on entend, au sens de l'invention, une quantité telle que le rapport molaire de l'acide de Lewis au dérivé benzénique azoté et chloré de départ est compris entre 0,0001 et 1 et de préférence entre 0,01 et 0,5.

Le procédé selon l'invention s'effectue en phase liquide (hormis bien sûr le catalyseur à base de métal noble). Cette phase liquide est organique et essentiellement anhydre. Elle est de préférence homogène, le milieu pouvant être constitué par le produit benzénique, azoté et chloré de départ, à l'état fondu (pour Y = H) ou par une solution de ce produit dans un solvant organique essentiellement anhydre et inerte dans les conditions de la réaction, solvant de préférence à point d'ébullition élevé, tel qu'un solvant aliphatique comme le cyclohexane, le dodécane ou un solvant aromatique tels que le chlorobenzène et les polychlorobenzènes. Ces derniers, en particulier, ne sont pas hydrochlorés dans les conditions de la réaction. La phase liquide peut également être hétérogène et constituée par un milieu liquide biphasé.

La pression, à laquelle s'effectue la réaction, peut être choisie dans une large gamme. Bien que cela ne soit pas avantageux, on peut opérer sous pression réduite, par exemple en milieu solvant à reflux. Cependant, de préférence, on travaille à une pression au moins égale à la pression atmosphérique. Le travail à pression atmosphérique permet d'apporter l'hydrogène sous forme de courant à un débit réglable.

Lorsqu'on travaille sous pression, la pression est généralement supérieure à 3 bars (pression relative) et de préférence supérieure à 5 bars. Il n'y a pas de limite supérieure critique pour la pression mais pour des raisons d'ordre éconimique, il est avantageux d'opérer à des pressions inférieures à 100 bars, les pressions inférieures à 20 bars étant préférées.

La température de réaction est généralement comprise entre 90 et 300°C, de préférence entre 110 et 200°C. Une température élevée peut, dans le cas où on utilise des acides de Lewis relativement volatils, impliquer l'existence d'une pression partielle relativement importante pour les composés de la phase vapeur autres que l'hydrogène (par phase vapeur on entend évidemment la phase vapeur surmontant le milieu liquide réactionnel).

Les métaux nobles constituant la base des catalyseurs utilisés dans l'invention sont des métaux du groupe VIII de la classification périodique tels que le ruthénium, le rhodium, le palladium, l'osmium, l'iridium et le platine; le palladium est le métal préféré. Le métal peut être à l'état métallique ou à l'état de composé chimique; généralement on préfère que le métal soit mis en oeuvre à l'état métallique.

Le catalyseur peut être supporté ou non supporté. Comme support du catalyseur, on peut utiliser tout support connu en soi pour supporter des catalyseurs, pourvu que ce support soit résistant au milieu et aux acides; comme support convenant plus particulièrement, on peut citer le charbon actif, l'alumine, la silice, le sulfate de baryum; le charbon actif est un support préféré. Le catalyseur ainsi que son support sont avantageusement sous forme finement divisée; des surfaces spécifiques supérieures à 100 $m^2/g$ conviennent généralement bien.

La quantité de catalyseur mise en oeuvre est telle que la proportion pondérale de métal noble du catalyseur par rapport au composé de formule (I) à traiter est généralement comprise entre 0,01 et 10%, de préférence entre 0,1 et 5%.

Par ailleurs, le métal noble peut être associé à un autre métal codéposé avec lui sur le support. Ce second métal appartient aux groupes 1b à 5a de la classification périodique. On peut citer en particulier le bismuth, le plomb, l'étain, le thallium, le mercure et l'argent. La demanderesse a notamment constaté que de bons résultats sont obtenus en utilisant l'argent.

Comme composés de formule (I) susceptibles d'être traités par le procédé de l'invention, on peut citer de préférence:

- le dichloro-2,3 nitrobenzène et la dichloro-2,3 aniline;
- le dichloro-2,5 nitrobenzène et la dichloro-2,5 aniline;
- le trichloro-3,4 nitrobenzène et la dichloro-3,4 aniline;
- le trichloro-2,3,4 nitrobenzène et la trichloro-2,3,4 aniline;
- le trichloro-2,3,5 nitrobenzène et la trichloro-2,3,5 aniline;
- le trichloro-2,3,6 nitrobenzène et la trichloro-2,3,6 aniline;
- le trichloro-2,4,5 nitrobenzène et la trichloro-2,4,5 aniline;
- le trichloro-3,4,5 nitrobenzène et la trichloro-3,4,5 aniline;
- le tétrachloro-2,3,4,6 nitrobenzène et la tétrachloro-2,3,4,6 aniline;
- le tétrachloro-2,3,4,5 nitrobenzène et la tétrachloro-2,3,4,5 aniline;
- le tétrachloro-2,3,5,6 nitrobenzène et la tétrachloro-2,3,5,6 aniline;
- le pentachloro-nitrobenzène et la pentachloroaniline; mais aussi:
- le trichloro-4,5,6 méthyl-2 nitrobenzène et la trichloro-4,5,6 méthyl-2 aniline;
- le dichloro-2,5 méthyl-4 nitrobenzène et la dichloro-2,5 méthyl-4 aniline;
- le tétrachloro-2,3,5,6 méthyl-4 nitrobenzène et la tétrachloro-2,3,5,6 méthyl-4 aniline;
- le dichloro-2,5 diméthyl-3,4 nitrobenzène et la dichloro-2,5 diméthyl-3,4 aniline;
- le dichloro-2,5 éthyl-4 nitrobenzène et la dichloro-2,5 éthyl-4 aniline;
- le dichloro-2,5 propyl-4 nitrobenzène et la dichloro-2,5 propyl-4 aniline;
- le trichloro-3,4,6 benzyl-2 nitrobenzène et la trichloro-3,4,6 benzyl-2 aniline;
- le dinitro-2,2′ hexachloro 3,5,6, 3′, 5′, 6′ diphénylméthane et le diamino-2,2′ hexachloro-3,5,6, 3′,5′,6′ diphénylméthane;
- le nitro-2 trichloro-3,4,5 diphényle et l'amino-2 trichloro-3,4,5 diphényle;
- le dinitro-4,4′ octachlorodiphényle et le diamino-4,4′ octachloro diphényle;
- le dichloro-4,5 méthoxy-2 nitrobenzène et la dichloro-4,5 méthoxy-2 aniline;
- le dichloro-3,4 méthoxy-2 nitrobenzène et la dichloro-3,4 méthoxy-2 aniline;
- le dichloro-3,6 méthoxy-2 nitrobenzène et la dichloro-3,6 méthoxy-2 aniline;
- le dichloro-5,6 méthoxy-2 nitrobenzène et la dichloro-5,6 méthoxy-2 aniline;
- le trichloro-3,4,6 méthoxy-2 nitrobenzène et la trichloro-3,4,6 méthoxy-2 aniline;
- le trichloro-3,4,5 méthoxy-2 nitrobenzène et la trichloro-3,4,5 méthoxy-2 aniline;
- le tétrachloro-3,4,5,6 méthoxy-2 nitrobenzène et la tétrachloro-3,4,5,6 méthoxy-2 aniline;
- le dichloro-4,5 méthoxy-3 nitrobenzène et la dichloro-4,5 méthoxy-3 aniline;
- le dichloro-5,6 méthoxy-3 nitrobenzène et la dichloro-5,6 méthoxy-3 aniline;
- le dichloro-2,5 méthoxy-3 nitrobenzène et la dichloro-2,5 méthoxy-3 aniline;
- le trichloro-4,5,6 méthoxy-3 nitrobenzène et la trichloro-4,5,6 méthoxy-3 aniline;
- le tétrachloro-2,4,5,6 méthoxy-3 nitrobenzène et la tétrachloro-2,4,5,6 méthoxy-3 aniline;
- le dichloro-2,3 méthoxy-4 nitrobenzène et la dichloro-2,3 méthoxy-4 aniline;
- le dichloro-2,5 méthoxy-4 nitrobenzène et la dichloro-2,5 méthoxy-4 aniline;
- le trichloro-2,3,6 méthoxy-4 nitrobenzène et la trichloro-2,3,6 méthoxy-4 aniline;
- le trichloro-2,3,5 méthoxy-4 nitrobenzène et la trichloro-2,3,5 méthoxy-4 aniline;
- le tétrachloro-2,3,5,6 méthoxy-4 nitrobenzène et la tétrachloro-2,3,5,6 méthoxy-4 aniline;
- le dichloro-4,5 phénoxy-2 nitrobenzène et la dichloro-4,5 phénoxy-2 aniline;
- le tétrachloro-3,4,5,6 phénoxy-2 nitrobenzène

et la tétrachloro-3,4,5,6 phénoxy-2 aniline;

- le tétrachloro-2,4,5,6 phénoxy-3 nitrobenzène et la tétrachloro-2,4,5,6 phénoxy-3 aniline;

- le dichloro-2,5 phénoxy-4 nitrobenzène et la dichloro-2,5 phénoxy-4 aniline;

- le tétrachloro-2,3,5,6 phénoxy-4 nitrobenzène et la tétrachloro-2,3,5,6 phénoxy-4 aniline.

Parmi les anilines substituées en position méta par l'atome de chlore et susceptibles d'être préparées par le procédé selon l'invention, on peut citer de préférence: la métachloraniline; la dichloro-3,5 aniline mais aussi la chloro-5 méthyl-2 aniline; la chloro-5 méthyl-3 aniline; la chloro-3 méthyl-4 aniline; la dichloro-3,5 méthyl-4 aniline; la chloro-5 diméthyl-3,4 aniline; la chloro-3 éthyl-4 aniline; la chloro-3 benzyl-2 aniline; le diamino-4,4' tétrachloro-2,6,2',6' diphényle; la chloro-3 méthoxy-2 aniline; la chloro-5 méthoxy-2 aniline; la dichloro-3,5 méthoxy-2 aniline; la chloro-3 méthoxy-4 aniline; la chloro-5 méthoxy-3 aniline; la dichloro-3,5 méthoxy-4 aniline; la chloro-3 phénoxy-2 aniline; la chloro-5 phénoxy-2 aniline; la dichloro-3,5 phénoxy-2 aniline; la dichloro-3,5 phénoxy-4 aniline.

Le procédé selon l'invention peut être effectué en continu ou en discontinu. En fin de réaction, le catalyseur peut être séparé, le cas échéant, par filtration ou par des moyens équivalents tels que l'essorage; l'aniline métachlorée préparée peut être séparée par tout moyen connu en soi, par exemple par extraction à l'aide d'un solvant et/ou par distillation.

Le procédé selon l'invention est très avantageux car il permet d'obtenir des anilines métachlorées dans d'excellentes conditions de sélectivité, à des températures et sous des pressions modérées, et ce sans souci de corrosion importante et d'usure prématurée du matériel.

Les exemples suivants, donnés à titre non limitatif illustrent la mise en oeuvre du procédé selon l'invention et les résultats obtenus.

Exemple 1

Dans un autoclave de 70 $cm^3$ revêtu intérieurement de tantale on charge:

- 1,16 g de tétrachloro-2,3,4,5 aniline,
- 0,4 g d'un catalyseur constitué de palladium déposé sur du charbon actif (surface spécifique du noir de carbone: 1300 $m^2/g$; teneur pondérale en palladium 10%),
- 17 $cm^3$ de cyclohexane anhydre,
- 3 $cm^3$ d'une solution à 5% (poids/volume) de bromure d'aluminium ($Al\ Br_3$) dans le cyclohexane anhydre.

L'autoclave est fermé, purgé d'abord à l'argon, puis à l'hydrogène. On introduit ensuite de l'hydrogène sous une pression de 10 bars, on isole l'autoclave et chauffe à 160°C en laissant croître la pression autogène jusqu'à une pression totale (relative) de 21 bars. On laisse réagir dans ces conditions pendant 4H30mn.

On refroidit. On filtre le contenu de l'autoclave pour séparer le catalyseur solide de la solution hexanique. Cette dernière est ensuite lavée par une solution aqueuse de soude (NaOH) de façon à neutraliser totalement l'acide chlorhydrique issu de la réaction.

Après distillation du cyclohexane, on obtient la 3,5-dichloraniline avec un rendement de 99,5 %.

Exemple 2

On opère comme à l'exemple 1 avec les charges suivantes:

- 1,16 g de 2,3,4,5 tétrachloraniline,
- 0,4 g d'un catalyseur constitué de palladium déposé à raison de 5% en poids sur le même charbon actif qu'à l'exemple 1,
- 0,095 g de chlorure stanneux anhydre (soit 0,082 partie par partie de TTCA),
- 20 $cm^3$ de cyclohexane.

On obtient la 3,5-dichloraniline avec un rendement de 96,5%.

Exemple 3

On opère comme à l'exemple 1 avec les charges suivantes:

- 1 g de 2,3,4,5-tétrachloraniline,
- 0,3 g du catalyseur utilisé à l'exemple précédent,
- 0,64 g de chlorure stanneux anhydre,

Ce dernier est dissout dans l'aniline fondue, à la température de réaction (160°C). La réaction dure 3H20mn.

Dans ces conditions, on obtient la 3,5-dichloraniline avec un rendement de 100%.

Exemple 4

On opère come à l'exemple 2 en remplaçant le chlorure stanneux par 0,32 g d'iodure de zinc. La réaction dure 10 heures.

Dans ces conditions, on obtient la 3,5-dichloraniline avec un rendement de 98,2%.

Exemples 5 à 13

Dans un autoclave de 70 $cm^3$ revêtu intérieurement de tantale, on charge:

- 1 g de 2,3,4,5-tétrachloraniline (TTCA),
- 0,3 g d'un catalyseur à 3% de palladium et 2% d'argent codéposés sur du charbon actif de surface spécifique d'environ 1300 $m^2/g$,
- une quantité variable d'un acide de Lewis comme catalyseur.

La réaction s'effectue à 160°C sous 21 bars. Le traitement du milieu réactionnel après réaction est effectué comme à l'exemple 1.

Le tableau ci-apres indique la durée de la réaction et le rendement en 3,5-dichloraniline pour chaque acide de Lewis et pour un rapport molaire acide de Lewis/tétrachloraniline (A.L/TTCA) donné.

| EX | Acide de Lewis | A.L/TTCA | Durée | Rendement en 3,5-DCA |
|---|---|---|---|---|
| 5 | $SnCl_2$ | 1,0 | 3H | 100% |
| 6 | $SnCl_4$ | 1,0 | 13H | 100% |
| 7 | $AlCl_3$ | 0,6 | 3HO5mn | 96,5% |
| 8 | $AlCl_3$ | 0,2 | 9H20mn | 92,5% |
| 9 | $AlBr_3$ | 0,02 | 6H15mn | 100% |
| 10 | $FeCl_3$ | 1,0 | 13H | 100% |
| 11 | $ZnCl_2$ | 1,0 | 9H20mn | 95,0% |
| 12 | CuCl | 1,0 | 10H40mn | 100% |
| 13 | $NiCl_2$ | 1,0 | 15H50mn | 100% |

Exemple 14

On opère comme à l'exemple 1 en remplaçant la 2,3,4,5-tétrachloraniline par une quantité égale de 2,3,5,6-tétrachloraniline. La réaction dure 13 heures.

Dans ces conditions, on obtient la 3,5-dichloroaniline avec un rendement de 99,6%.

Exemple 15

On opère comme à l'exemple 1 en remplaçant la 2,3,4,5-tétrachloraniline par la 3,4,5-trichloraniline (0,985 g), l'acide de Lewis étant ajouté sous forme de 1 $cm^3$ d'une solution cyclohexanique à 10% p/v et en utilisant 0,05 g du même catalyseur au palladium.

La réaction est conduite pendant 120 mn.

Dans ces conditions, on obtient la 3,5-dichloraniline avec un rendement de 86%.

Exemple 16

Dans un réacteur de 250 ml, équipé d'un agitateur central tournant à 1000 tours/mn, d'un réfrigérant, d'un thermomètre et d'une arrivée de gaz, on charge:

- 10 g (0,05 mole) de trichloro-3,4,5-aniline,
- 0,4 g (1,25 × $10^{-3}$ mole) de iodure de zinc ($ZnI_2$),
- 0,5 g d'un catalyseur au palladium à 5% sur charbon actif,
- 56,5 g (0,35 mole) de dichloro-3,5 aniline.

On introduit ensuite l'hydrogène comme à l'exemple 1, si ce n'est que la pression totale est de 20 bars et que le chauffage est effectué à 180°C pendant deux heures.

Dans ces conditions, toute la trichloro-3,4,5-aniline est tranformée et on obtient la 3,5-aniline avec un rendement de 100%.

Exemple 17

Dans un ballon de capacité 100 ml, muni d'un agitateur central tournant à 1000 tours/mn, d'un réfrigérant, d'un thermomètre et d'une arrivée de gaz, on charge:

- 2 g (environ 0,01 mole) de trichloro-3,4,5-aniline,
- 0,6 g (2,25 × $10^{-3}$ mole) de bromure d'aluminium ($AlBr_3$),
- 0,8 g d'un catalyseur au palladium à 5% sur charbon actif,
- 20 ml de dodécane.

On maintient une température de 160°C et à la pression atmosphérique on envoie un courant d'hydrogène de 6 litres/h pendant 30 mn.

La masse réactionnelle est ensuite traitée par de l'eau puis de l'éther. On sépare le catalyseur puis l'eau. On élimine les solvants par distillation.

Dans ces conditions, on obtient la dichloro-3,5-aniline avec un rendement de 99,5%. Le taux de transformation de la trichloro-3,4,5 aniline est de 100%.

Exemple 18

On opère comme à l'exemple précédent dans un réacteur de 250 ml, équipé de la même manière, avec les charges suivantes:

- 19,7 g (0,1 mole) de trichloro-3,4,5-aniline,
- 3,0 g (1,12 × $10^{-2}$ mole) de bromure d'aluminium ($AlBr_3$),
- 0,5 g du catalyseur au palladium à 5% sur charbon actif,
- 60 ml de trichloro-1,2,4-benzène.

Le milieu réactionnel étant mis sous agitation, on maintient une température de 160°C et un courant d'hydrogène de 27 l/h pendant 4 heures.

En opérant comme à l'exemple précédent, on obtient la dichloro-3,5 aniline avec un rendement de 100%.

**Revendications**

1. Procédé de préparation d'anilines substituées en position méta par du chlore, par hydrogénation catalytique en phase liquide, à chaud à la pression atmosphérique ou sous pression, en présence de métaux nobles du groupe VIII de la classification périodique, de dérivés benzéniques azotés et chlorés, de formule

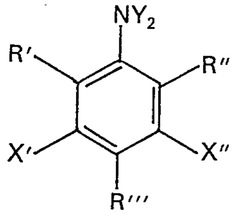

(I)

dans laquelle:

- Y représente l'atome d'hydrogène ou l'atome d'oxygène,
- X′ et X″, identiques ou différents entre eux, représentent chacun un atome de chlore ou un radical alkyle ou aryle ou aralkyle ou alkoxyle ou

aralkoxyle éventuellement substitué, au moins l'un des X' et X" étant obligatoirement un atome de chlore, l'un des X' et X" pouvant, de plus, être l'hydrogène,

- R', R" et R"', identiques ou différents entre eux, représentent chacun un atome de chlore ou un radical alkyle ou aryle ou aralkyle ou alkoxyle ou aryloxyle éventuellement substitué, l'un au moins de ces trois symboles représentant l'atome de chlore, au plus deux des R', R" ou R"' pouvant, de plus, être l'hydrogène,

caractérisé en ce que la réaction est effectuée en milieu essentiellement anhydre, en présence d'une quantité catalytique d'au moins un acide de Lewis comme catalyseur.

2. Procédé selon la revendication 1, caractérisé en ce que l'acide de Lewis est un halogénure d'un élément choisi dans le groupe comprenant le bore, l'aluminium, le gallium, l'étain, le phosphore, l'arsenic, l'antimoine, le bismuth, le titane, le zirconium, le molybdène, le manganèse, le fer, le cobalt, le nickel, le cuivre, le zinc et le cadmium.

3. Procédé selon la revendication 2, caractérisé en ce que la réaction est effectuée en présence d'une quantité catalytique d'un composé choisi dans le groupe comprenant les chlorure et bromure d'aluminium, les chlorures stanneux et stannique, le chlorure ferrique, les chlorure et iodure de zinc, le chlorure cuivreux et le chlorure de nickel.

4. Procédé selon l'une des revendications 1 à 3, caractérisé en ce que le rapport molaire de l'acide de Lewis au dérivé benzénique azoté et chloré de départ est compris entre 0,0001 et 1.

5. Procédé selon la revendication 4, caractérisé en ce que le rapport molaire de l'acide de Lewis au dérivé benzénique azoté et chloré de départ est compris entre 0,01 et 0,5.

6. Procédé selon l'une des revendications 1 à 5, caractérisé en ce que la réaction est effectuée dans le produit de départ fondu.

7. Procédé selon l'une des revendications 1 à 5, caractérisé en ce que la réaction est effectuée en solution dans un solvant organique.

8. Procédé selon l'une des revendicatios 1 à 7, caractérisé en ce que R', R", R"', X', X", identiques ou différents entre eux, représentent l'atome d'hydrogène ou l'atome de chlore.

9. Procédé de préparation de dimétachloroanilines, éventuellement substituées, selon l'une des revendications 1 à 8, caractérisé en ce que X' et X" représentent l'atome de chlore.

10. Procédé de préparation de monométachloroanilines, éventuellement substituées, selon l'une des revendications 1 à 8, caractérisé en ce que un seul des deux radicaux X' et X" est l'atome de chlore.

11. Procédé de préparation de dichloro-3,5 aniline selon les revendications 1 à 9, caractérisé en ce que:

- Y est l'atome d'hydrogène ou d'oxygène,
- X' et X" sont l'atome de chlore,
- R', R", R"' sont l'atome d'hydrogène ou l'atome de chlore, l'un d'entre eux au moins étant l'atome de chlore.

12. Procédé selon l'une des revendications 1 à 11, caractérisé en ce que le milieu réactionnel ne comporte qu'une phase liquide, hormis le catalyseur à base de métal noble.

13. Procédé selon l'une des revendications 1 à 12, carctérisé en ce que la réaction est effectuée à une pression inférieure à 3 bars.

14. Procédé selon la revendication 13, caractérisé en ce que la réaction est effectuée à la pression atmosphérique.

15. Procédé selon l'une des revendications 1 à 12, caractérisé en ce que la pression totale est comprise entre 3 et 100 bars.

16. Procédé selon la revendication 15, caractérisé en ce que la pression totale est comprise entre 5 et 20 bars.

17. Procédé selon l'une des revendications 1 à 16, caractérisé en ce que la température est comprise entre 90 et 300°C, de préférence entre 110 et 200°C.

18. Procédé selon l'une des revendications 1 à 17, caractérisé en ce que la catalyseur est le palladium.

19. Procédé selon l'une des revendications 1 à 18, caractérisé en ce que la proportion pondérale de métal noble par rapport au composé de formule (I) est comprise entre 0,01 et 10%, de préférence entre 0,1 et 5%.

**Patentansprüche**

1. Verfahren zur Herstellung m-chlorsubstituierter Aniline durch katalytische Hydrierung von stickstoffhaltigen und chlorierten Benzolderivaten der Formel

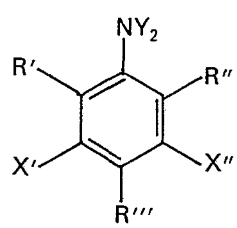

(I)

in der bedeuten:

Y Wasserstoff oder Sauerstoff,

X' und X" zugleich oder unabhängig jeweils Chlor oder Alkyl, Aryl, Aralkyl, Alkoxy oder Aralkoxy, die jeweils gegebenenfalls substituiert sein können, wobei mindestens einer der Substituenten X' und X" zwingend Chlor ist und einer der Substituenten X' und X" ferner Wasserstoff bedeuten kann,

und

R', R" und R"' zugleich oder unabhängig jeweils Chlor oder Alkyl, Aryl, Aralkyl, Alkoxy oder Aryloxy, die gegebenenfalls substituiert sein können, wobei mindestens einer dieser Substituenten Chlor darstellt und höchstens zwei der Substituenten R', R" und R"' ferner Wasserstoff bedeuten können, in flüssiger Phase in der Wärme bei Atmosphärendruck oder unter Druck in Gegenwart von Edelmetallen der Gruppe VIII des Periodensystems, dadurch gekennzeichnet, dass die Reaktion in einem im wesentlichen wasserfreien Medium in Gegenwart einer katalytischen Menge mindestens einer Lewissäure als Katalysator durchgeführt wird.

2. Verfahren nach Anspruch 1, dadurch gekennzeichnet, dass als Lewissäure ein Halogenid von Bor, Aluminium, Gallium, Zinn, Phosphor, Arsen, Antimon, Wismut, Titan, Zirkonium, Molybdän, Mangan, Eisen, Kobalt, Nickel, Kupfer, Zink und/oder Cadmium verwendet wird.

3. Verfahren nach Anspruch 2, dadurch gekennzeichnet, dass die Reaktion in Gegenwart einer katalytischen Menge einer unter Aluminiumchlorid, Aluminiumbromid, Zinn(II)chlorid, Zinn(IV)chlorid, Eisen(III)chlorid, Zinkchlorid, Zinkjodid, Kupfer(I)chlorid und Nickelchlorid ausgewählten Verbindung durchgeführt wird.

4. Verfahren nach einem der Ansprüche 1 bis 3, dadurch gekennzeichnet, dass ein Molverhältnis von 0,0001 bis 1 angewandt wird.

5. Verfahren nach Anspruch 4, dadurch gekennzeichnet, dass ein Molverhältnis von Lewissäure zur als Ausgangsmaterial eingesetzten Verbindung der Formel I 0,01 bis 0,5 angewandt wird.

6. Verfahren nach einem der Ansprüche 1 bis 5, dadurch gekennzeichnet, dass die Reaktion in einer Schmelze der als Ausgangsmaterial eingesetzten Verbindung durchgeführt wird.

7. Verfahren nach einem der Ansprüche 1 bis 5, dadurch gekennzeichnet, dass die Reaktion in Lösung in einem organischen Lösungsmittel durchgeführt wird.

8. Verfahren nach einem der Ansprüche 1 bis 7, dadurch gekennzeichnet, dass ein Benzolderivat der Formel I verwendet wird, in dessen Formel R′, R″, R‴, X′ und X″ zugleich oder unabhängig Wasserstoff oder Chlor bedeuten.

9. Verfahren zur Herstellung von gegebenenfalls substituierten Di-m-chloranilinen nach einem der Ansprüche 1 bis 8, dadurch gekennzeichnet, dass ein Benzolderivat der Formel I verwendet wird, in der X′ und X″ Chlor bedeuten.

10. Verfahren zur Herstellung gegebenenfalls substituierter Mono-m-chloraniline nach einem der Ansprüche 1 bis 8, dadurch gekennzeichnet, dass ein Benzolderivat der Formel I verwendet wird, in der einer der Substituenten X′ und X″ Chlor bedeutet.

11. Verfahren zur Herstellung von 3,5-Dichloranilin nach einem der Ansprüche 1 bis 9, dadurch gekennzeichnet, dass ein Benzolderivat der Formel I verwendet wird, in der Y Wasserstoff oder Sauerstoff, X′ und X″ jeweils Chlor und R′, R″ und R‴ Wasserstoff oder Chlor bedeuten, wobei mindestens einer dieser Substituenten Chlor ist.

12. Verfahren nach einem der Ansprüche 1 bis 11, dadurch gekennzeichnet, dass ein Reaktionsmedium verwendet wird, das ausser dem Katalysator auf der Basis des Edelmetalls nur eine flüssige Phase aufweist.

13. Verfahren nach einem der Ansprüche 1 bis 12, dadurch gekennzeichnet, dass die Reaktion bei einem Druck niedriger als 3 bar durchgeführt wird.

14. Verfahren nach Anspruch 13, dadurch gekennzeichnet, dass die Reaktion bei Atmosphärendruck durchgeführt wird.

15. Verfahren nach einem der Ansprüche 1 bis 12, dadurch gekennzeichnet, dass die Reaktion bei einem Gesamtdruck von 3 bis 100 bar durchgeführt wird.

16. Verfahren nach Anspruch 15, dadurch gekennzeichnet, dass die Reaktion bei einem Gesamtdruck von 5 bis 20 bar durchgeführt wird.

17. Verfahren nach einem der Ansprüche 1 bis 16, dadurch gekennzeichnet, dass bei einer Temperatur von 90 bis 300°C vorzugsweise von 110 bis 200°C gearbeitet wird.

18. Verfahren nach einem der Ansprüche 1 bis 17, dadurch gekennzeichnet, dass als Katalysator Palladium verwendet wird.

19. Verfahren nach einem der Ansprüche 1 bis 18, dadurch gekennzeichnet, dass das Edelmetall in einer Menge von 0,01 bis 10 Gew.-%, vorzugsweise von 0,1 bis 5 Gew.-%, bezogen auf die Verbindung der Formel I, verwendet wird.

**Claims**

1. A process for the preparation of anilines substituted in the meta-position by chlorine, by the catalytic hydrogenation, in the liquid phase, under the action of heat and at atmospheric pressure or under pressure, in the presence of noble metals from group VIII of the periodic classification, of nitrogen-containing and chlorine-containing benzene derivatives of the formula:

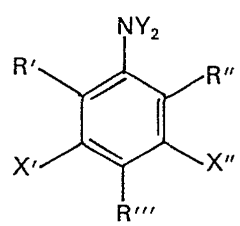

(I)

in which: Y represents the hydrogen atom or the oxygen atom, X′ and X″, which are identical to or different from one another, each represent a chlorine atom, or an optionally substituted alkyl, aryl, aralkyl, alkoxy or aralkoxy radical, at least one of the symbols X′ and X″ necessarily being a chlorine atom and it being furthermore possible for one of the symbols X′ and X″ to be hydrogen, and R′, R″ and R‴, which are identical to or different from one another, each represent a chlorine atom or an optionally substituted alkyl, aryl, aralkyl, alkoxy or aryloxy radical, at least one of these three symbols representing the chlorine atom and it being furthermore possible for at most two of the symbols R′, R″ or R‴ to be hydrogen, in which process the reaction is carried out in an essentially anhydrous medium, in the presence of a catalytic amount of at least one Lewis acid as a catalyst.

2. A process according to claim 1, wherein the Lewis acid is a halide of an element chosen from the group comprising boron, aluminium, gallium, tin, phosphorus, arsenic, antimony, bismuth, titanium, zirconium, molybdenum, manganese, iron, cobalt, nickel, copper, zinc and cadmium.

3. A process according to claim 2, wherein the reaction is carried out in the presence of a catalytic amount of a compound chosen from the group comprising aluminium chloride and bromide, stannous and stannic chlorides, ferric chloride, zinc chloride and iodide, cuprous chloride and nickel chloride.

4. A process according to one of claims 1 to 3, wherein the molar ratio of the Lewis acid to the starting nitrogen-containing and chlorine-containing benzene derivative is between 0.0001 and 1.

5. A process according to claim 4, wherein the molar ratio of the Lewis acid to the starting nitrogen-containing and chlorine-containing benzene derivative is between 0.01 and 0.5.

6. A process according to one of claims 1 to 5, wherein the reaction is carried out in the molten starting material.

7. A process according to one of claims 1 to 5, wherein the reaction is carried out in solution in an organic solvent.

8. A process according to one of claims 1 to 7, wherein R′, R″, R‴, X′ and X″, which are identical to or different from one another, represent the hydrogen atom or the chlorine atom.

9. A process for the preparation of optionally substituted meta-dichloroanilines, according to one of claims 1 to 8, wherein X′ and X″ represent the chlorine atom.

10. A process for the preparation of optionally substituted meta-monochloroanilines, according to one of claims 1 to 8, wherein only one of the two radicals X′ and X″ is the chlorine atom.

11. A process for the preparation of 3,5-dichloroaniline, according to claims 1 to 9, wherein: Y is the hydrogen or oxygen atom, X′ and X″ are the chlorine atom and R′, R″ and R‴ are the hydrogen atom or the chlorine atom, at least one of them being the chlorine atom.

12. A process according to one of claims 1 to 11, wherein the reaction medium only contains a liquid phase, except for the catalyst based on a noble metal.

13. A process according to one of claims 1 to 12, wherein the reaction is carried out at a pressure below 3 bars.

14. A process according to claim 13, wherein the reaction is carried out at atmospheric pressure.

15. A process according to one of claims 1 to 12, wherein the total pressure is between 3 and 100 bars.

16. A process according to claim 15, wherein the total pressure is between 5 and 20 bars.

17. A process according to one of claims 1 to 16, wherein the temperature is between 90 and 300°C, preferably between 110 and 200°C.

18. A process according to one of claims 1 to 17, wherein the catalyst is palladium.

19. A process according to one of claims 1 to 18, wherein the proportion by weight of noble metal, relative to the compound of the formula (I), is between 0.01 and 10%, preferably between 0.1 and 5%.